(19) Europäisches Patentamt
European Patent Office
Office européen des brevets

(11) EP 1 207 911 B1

(12) **EUROPEAN PATENT SPECIFICATION**

(45) Date of publication and mention of the grant of the patent:
**19.04.2006 Bulletin 2006/16**

(21) Application number: **00956719.9**

(22) Date of filing: **01.09.2000**

(51) Int Cl.:
***A61K 51/12*** *(2006.01)*

(86) International application number:
**PCT/GB2000/003374**

(87) International publication number:
**WO 2001/017569 (15.03.2001 Gazette 2001/11)**

# (54) Composition comprising radiopharmaceutical metal complexes in silica-coated containers

Zusammensetzung von radiopharmazeutischen Metallkomplexen in Silika-beschichteten Behältnissen

Composition de complexes métalliques radiopharmaceutiques dans des contenants recouverts de silice

(84) Designated Contracting States:
**AT BE CH CY DE DK ES FI FR GB GR IE IT LI LU MC NL PT SE**

(30) Priority: **03.09.1999 GB 9920772**

(43) Date of publication of application:
**29.05.2002 Bulletin 2002/22**

(73) Proprietor: **GE Healthcare Limited**
**Little Chalfont**
**Buckinghamshire HP7 9NA (GB)**

(72) Inventors:
- **GILL, Stephen**
  **GE Healthcare Ltd.,The Grove Centre**
  **Amersham**
  **Buckinghamshire HP7 9LL (GB)**
- **RUSHOWSKI, Stanley Francis**
  **Chesham**
  **Buckinghamshire HP5 3BE (GB)**

(74) Representative: **Canning, Lewis R. et al**
**GE Healthcare Limited**
**Amersham Place**
**Little Chalfont,**
**Bucks. HP7 9NA (GB)**

(56) References cited:
**WO-A-96/26163**
**DE-A- 19 801 861**
**US-A- 5 565 248**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 207 911 B1

## Description

### Summary of the Invention

The present invention relates to improved containers for radiopharmaceutical metal complexes, where the container has an internal coating of silica (ie. silicon dioxide or $SiO_2$), preferably deposited by a plasma chemical vapour deposition (PCVD) process.

### Field of the Invention

The present invention relates to radiopharmaceutical metal complexes, and kits for the preparation of such complexes, in a container having a silica coating on the surface(s) which are in contact with the radiopharmaceutical.

### Background to the Invention

US 4385086 (1983) discloses that a variety of materials (eg. soda glass, ceramics and metals) can be coated with highly oxidised silicon to prevent the leaching of metal ions from the material.

FR 2697014 A1 (1994) discloses the silica coating of bottles, flasks, ampoules and other containers intended for use with food or liquid pharmaceutical products, to reduce leaching of metals from the container into the liquid contents of the container.

DE 29609958 U1 discloses that glass containers having an internal coating of $SiO_2$ prepared by PCVD are useful for the storage of pharmaceutical or diagnostic solutions.

JP 11-99192A discloses that silica-coated vials (prepared by a chemical coating and pyrolysis method), are useful to prevent adsorption of radiopharmaceutical products such as $^{201}$T1 solution to the surface of the glass. The silica coating of these vials is manufactured by the method described in JP 2815595 B which involves treating the glass surface with a silyl tetraisocyanate vapour in a carrier gas, followed by heating at high temperatures. JP 2815595 B also discloses that such a silica coating is useful to prevent leaching of impurities such as alkali from the glass into medical products.

### Summary of the Invention

The present invention relates to silica-coated containers in combination with the following categories of products:

(i) radioactive radiopharmaceutical products which are metal complexes,
(ii) lyophilised non-radioactive kits for the preparation of radiopharmaceutical metal complexes, especially for the preparation of $^{99m}$Tc radiopharmaceuticals.

### Detailed Description of the Invention

The present invention relates to a composition comprising a radiopharmaceutical metal complex in a container which has a silica coating on the inner surface. The present invention also relates to non-radioactive, preferably lyophilised kits for the preparation of radiopharmaceutical metal complexes, where the kit composition is supplied in a container which has a silica coating on the inner surface.

Suitable silica-coated containers for use in the present invention are commercially available, e.g. a silica-coated vial called Silicoat is available from Fuji Glass KK, and a silica-coated vial called Type I Plus is available from Schott Glas. The Type I Plus vial is prepared by a plasma chemical vapour deposition (PCVD) process.

Other containers can be coated with silica using known methods, where the silicon-containing layer is deposited from either gas phase or liquid phase contact with the container surface(s), with optional pyrolysis and/or oxidation to convert the deposited silicon-containing layer to $SiO_2$. Such methods are known in the art. Using either approach, irregularly-shaped containers can be coated. Examples of gas phase deposition are PCVD, and the process of JP 2815595 B which uses silyl tetraisocyanate vapour in carrier gas. The latter process delivers the silicon-containing layer in a single step, with pyrolysis of silyl tetraisocyanate required to give the final product, i.e. the coated vial. Depending on the efficiency of the heat transfer, the coating layer may not be pure $SiO_2$ but perhaps contain carbon or nitrogen. The silica-coated vial prepared by PCVD has advantages over the disclosures of JP 2815595 B and JP 11-99192A, because the $SiO_2$ layer prepared by PCVD is in fact developed by multiple exposure to the vapour phase silicon reagent. The result is a much more uniform layer of high purity $SiO_2$ which is mechanically sound and resistant to abrasion etc. Hence PCVD is a preferred process for use in containers of the present invention.

The term "metal complex" as used herein means a coordination complex of a metal (M) with an organic ligand

(L). This is to be contrasted with an uncomplexed or free metal ion e.g. the monovalent thallium cation $T1^+$. The term 'organic ligand' as used herein means a carbon-containing compound which comprises at least one heteroatom suitable for coordination to a metal, such as N, O, S, P or Se, or combinations thereof. Examples of organic ligands are amines, hydrazines (eg. hydrazinonicotinamide or HYNIC), ethers such as crown ethers, thiols or thioethers, oximes, isonitriles (eg. sestamibi), phosphines, amides, pyridines or other heterocyclic molecules such as quinolines. Multiple metal donor atoms can be arranged together to form chelating agents or polydentate ligands such as:

- diaminedioximes such as propyleneamineoxime (ie. PnAO), or hexamethylpropyleneamineoxime (ie. HMPAO), or analogues thereof;
- hydroxyquinolines;
- N2S2 ligands such as diaminedithiols (eg. ethylcysteineate dimer or ECD, ie. bicisate), diamidedithiols or amideaminedithiols;
- N3S ligands such as thioltriamides (eg. mercaptoacetyltriglycine or MAG3);
- diphosphines (eg. tetrofosmin);
- dithiols (eg. dimercaptosuccinic acid or DMSA);
- aminocarboxylate ligands (eg. EDTA or DTPA);

and many more combinations as are well known in the art.

If leached metal ions M' (such as aluminium or sodium), leach from glass into a chemical product which is a radiopharmaceutical metal complex of a radiometal M, these leached metal ions may adversely affect the product in a manner which goes beyond the simple presence of M' as an impurity:

(i)

$$ML_n + M' \rightarrow M'Lq + M \qquad \text{ligand exchange or transmetallation}$$

(ii)

$$L + M' \rightarrow M'Lq \qquad \text{complexation}$$

where:

M is the radiometal of the desired radiopharmaceutical product,
L is the organic ligand;
M' is the leached metal ion;
$ML_n$ is the metal complex radiopharmaceutical product, which may comprise 2 or more different organic ligands L;
n is number of ligands (L) attached to M and is an integer of value 1 to 8;
$M'L_q$ is the metal complex impurity;
q is number of ligands (L) attached to M' and is an integer of value 1 to 8.

Process (i) can occur when the leached metal or metals (M') have greater affinity for one or more of the organic ligands (L) than the radiometal (M) of the radiopharmaceutical product. For a radioactive product, $ML_n$ is the radiopharmaceutical and could be e.g. $^{111}In(oxine)_3$ where M is $^{111}In$ and oxine is 8-hydroxyquinoline, or the $^{99m}Tc$ complex of DMSA (dimercaptosuccinic acid). It is important to recognise that, for radiopharmaceutical products, $ML_n$ is present at extremely low (typically nanomolar or picomolar) concentrations. Consequently, even low levels of leached metal ions (M') from the glass, can have a significant effect on the radiochemical purity of the $ML_n$ product, by increasing the levels of free radiometal (M) impurity. Such free radiometal could then generate further radioactive impurities by undergoing e.g. redox reactions, or complexation with other available ligands.

In addition to, or instead of equation (i), complexation (ii) may also occur. This leads to the presence of undesirable $M'L_q$ impurities in the product $ML_n$. $M'L_q$ is non-radioactive, but for a radiopharmaceutical agent, the ligand (L) is usually present in vast chemical excess over the metal (M) present, hence if M' has any affinity for L, equation (ii) is always likely to occur. $M'L_q$ may be insoluble in the medium present in the radiopharmaceutical vial or container, and may thus tend to precipitate. This may potentially promote co-precipitation of the desired species $ML_n$, and hence introduce yet further potential radioactive impurities.

When L is a multidentate ligand, such as a chelating agent the number of metal donor sites (A) per ligand (L) may be 2, 3, 4, 5, 6 or 8 typically. In that case, a process which is a special case of equation (i) above could occur as follows:

(iii) 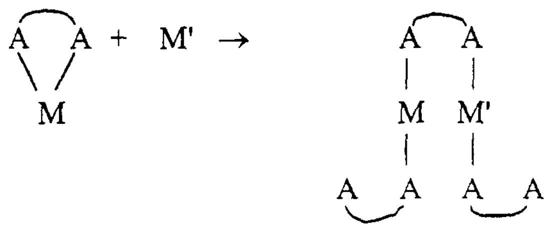

where: the free A donors can complex to further M/ M' atoms etc.
note: the curved lines represent the chain of atoms linking the A groups.
leading to dimeric or oligomeric binuclear or polynuclear metal complexes involving both radiometal M and non-radioactive metal M'. The leached metal (M') may be less amenable to chelation by polydentate ligand (L), and hence favour such polynuclear species, even when M does not. This would result when the energetics are less favourable, e.g. M' is too small for two A groups to coordinate without undue steric interactions from either the A groups themselves, or the ligand backbone linking the A groups. Clearly, the greater the denticity of the ligand L (i.e. the greater the number of A metal donor sites), the greater the potential complexity of the impurity product.

In the light of the above, it can be seen that the influence of leachable metal ions (M'), can have effects which go far beyond just the presence of M' as a metal ion impurity. This is important for radiopharmaceutical metal complex products and is not recognised by JP 11-99192A, which makes no specific reference to radiopharmaceuticals which are metal complexes. The $^{201}T1$ radioisotope taught by JP 11-99192 A is an uncomplexed radiometal in the chemical form of the T1(I) cation $T1^+$. The teaching of JP 11-99192 A relates only to adsorption effects *via* an ion exchange mechanism for the T1-201 cation (ie. $T1^+$) with the non-radioactive $Na^+$ and $K^+$ ions of the glass container walls. The main thrust of JP 11-99192 A is to a radiopharmaceutical vial having reversed text characters on the surface of the container

For uncoated glass containers, the leaching of metal ions from the glass can potentially be overcome, or at least reduced, by washing with dilute aqueous acid solutions (to remove relatively labile leachable metal ions), following by rinsing and (optionally) drying steps, before the container is charged with product. The layer of silica ($SiO_2$) suppresses any such leaching of metal ions (M'), and hence obviates the need for any such washing steps. This simplification is particularly important for diagnostic products intended for human use such as radiopharmaceuticals, which are typically administered by injection into the human bloodstream, since these washing steps must be done in a sterile manner. Hence although such steps may be straightforward, their removal represents a significant improvement.

The radiopharmaceutical product $^{111}$In-oxine aqueous solution has been observed to suffer from an intermittent problem whereby, in some batches $^{111}$In activity is lost to an insoluble precipitate which settles out of solution. Model experiments have identified this precipitate as a mixture of aluminium oxinate, with co-precipitated $^{111}In(oxine)_3$ (see Example 2). This illustrates the fact that the leaching of metal ions, in that case aluminium, from glass can cause unforeseen problems for metal complex radiopharmaceuticals, even when the initial effect is simply to form a $M'L_q$ complex as per equation (ii) above. It will be recognised that the interaction of leached metal ions with radiopharmaceutical metal complexes at tracer concentrations could produce a variety of impurities which are difficult to predict, identify and control when the glass can function as an uncontrolled source of a variety of metal ion impurities. Consequently, elimination of the leached metal ion by use of an inert barrier layer of $SiO_2$ eliminates the cause of this problem. Example 1 shows that a silica-coated vial reduces the leaching of aluminium to minimal levels, and hence solves the problem referred to above for $^{111}In(oxine)_3$.

Similarly, a lyophilised non-radioactive kit for the preparation of the kidney imaging agent $^{99m}$Tc-DMSA has been found to suffer from problems of radioactive particulate impurities following reconstitution with $^{99m}$Tc-pertechnetate ($^{99m}TcO_4^-$). The particle size of these radioactive particles is such that, once administered to the patient, the particles are retained in the liver. This represents a major problem for an agent which is intended to be used for kidney imaging. Example 4 shows that this biodistribution problem occurs when higher concentrations of aluminium ions than normal are present. Hence eliminating the leaching of aluminium from the glass vial is expected to significantly reduce or eliminate this problem. The problem with $^{99m}$Tc-DMSA indicates that leaching can occur even in lyophilised products which are reconstituted to give the radiopharmaceutical, i.e. that leaching can occur even when the contact time of the solution with the glass is only a matter of minutes.

JP 11-99192A concerns itself only with the issue of whether a free radioactive metal ion, ($^{201}T1$ as $T1^+$), is adsorbed onto the vial walls. For a radiopharmaceutical metal complex ($ML_n$), the non-radioactive organic ligand L and its complex $M'L_q$ may suffer from adsorption problems. L may also potentially complex with M' ions which remain bound within the container walls to form $M'L_q$ complexes chemically bound to the glass. Adsorption is particularly likely to be a problem when L and/or $M'L_q$ is not too soluble in the solution medium, e.g. a lipophilic ligand such as oxine in a predominantly aqueous medium. Such loss of ligand due to adsorption or complexation may not adversely affect the

preparation of the metal complex ($ML_n$) *per se,* since for a radiopharmaceutical L is usually present in vast chemical excess over M. Unwarranted loss of L does, however, impact on the reproducibility of manufacture of a controlled diagnostic product for human administration, since. variable assay results for L are highly undesirable with respect to GMP. Example 3 demonstrates oxine ligand loss problems for an oxine ligand aqueous solution formulation, and shows that loss of oxine can occur by both the mechanisms referred to above. Silica-coated vials would eliminate loss of oxine to aluminium, and would be expected to reduce the degree of adsorption. The overall effect would be a reduction in the loss of oxine ligand from the solution. Hence, for these reasons also, metal complex radiopharmaceuticals in silica-coated vials have surprising advantages compared to what is described in the prior art.

The silica-coated vial can be used in combination with other non-radioactive components of a kit for the preparation of metal complex radiopharmaceuticals apart from the organic ligand (L), e.g. stabilisers, or bacteriostats. Such stabilisers or bacteriostats may suffer from analogous problems to those described above for oxine - ie. potential adsorption and/or complexation with leached metals. Bacteriostats include parabens, benzyl alcohol, cetrimide, benzoic acid, chlorbutanol, chlorocresol, cresol, phenol, benzethonium chloride and thiomersal. Some bacteriostats such as methyl paraben (ie. methyl p-hydroxybenzoate), or propyl paraben (propyl *p*-hydroxybenzoate) are relatively insoluble in water, and hence a silica-coated container would be expected to reduce formulation problems. Also, cobalt is known as a stabiliser for the $^{99m}$Tc metal complex of the ligand HMPAO (hexamethylpropyleneamine oxime) [Eur. J. Nucl. Med., 20, 661-6 (1993) P. S. Weisner *et al*], and may be supplied in a separate vial to the HMPAO ligand vial. Reduced adsorption of cobalt onto the vial walls of a silica-coated vial is shown in Example 5. This shows that adsorption can occur in macroscopic amounts, not just at the tracer concentration described in the prior art for $^{201}$T1, and that silica-coated containers are useful for excipients used in the preparation of metal complex radiopharmaceuticals, such as stabilisers or bacteriostats.

Experimental

Example 1

Groups of 10 Type I Plus vials (Schott Glas) were subjected to a series of stress tests to demonstrate the robustness of the silica coating with respect to leachable ions.

The basic test was the resistance of the coating to the leaching of cations when autoclaved with 0.04M aqueous HCl. This test was performed after vials were exposed to the following stress test conditions 1 to 4:

1. Vials were washed, and then pyrogen baked. 2ml of 0.04M HCl was added and the vials sealed. Test vials were autoclaved, then stored upright at 40°C before testing for leachable cations.
2. Vials were stored for 6 weeks at -196°C, then washed and pyrogen baked. 2ml of 0.04M HCl was added to each vial, and the vials were then sealed, autoclaved and tested for leachable cations.
3. As test 2, except that the vials were stored at -70°C, -20°C, +20°C and +40°C/75% relative humidity.
4. Further tests included vials pyrogen baked 3 times, vials containing 0.04M HCl autoclaved three times, vials gamma irradiated (35.4 - 36.2 kGy dose).

All test solutions were measured by ICP for silicon, sodium, aluminium and boron, ie. those cations considered to be most leachable from the vial surface. The results are given in Table 1.

Table 1

| Test Number | | Si | Na | Al | B |
|---|---|---|---|---|---|
| 1 | | 0.149 | Nd | 0.006 | Nd |
| 2 | | 0.163 | Nd | Nd | Nd |
| 3 | -70°C | 0.167 | Nd | Nd | Nd |
| | -20°C | 0.193 | 0.005 | 0.002 | 0.002 |
| | +20°C | 0.193 | 0.009 | 0.005 | 0.003 |
| | +40°C | 0.236 | 0.006 | 0.002 | 0.002 |
| 4 | bake | 0.110 | Nd | 0.010 | Nd |
| | X3 | 0.378 | 0.012 | Nd | 0.006 |
| | Gamma | 0.102 | 0.003 | Nd | Nd |

Note: each table entry is the mean of 12 batch runs, each batch of 10 vials (i.e. 120 vials tested), expressed in μg/$cm^3$ of test solution.
Nd = not detected. Detection limits (in μg/$cm^3$):

Si - 0.003
Na - 0.004
Al - 0.004
B - 0.004

All of the results were satisfactory, particularly for the key cations sodium and aluminium, each of which had mean values of approximately 0.01μg per ml of test solution. These very low levels demonstrate the robustness of the silica coating under stress conditions.

There were no significant differences in the results obtained between vials from different proving runs. This demonstrates the reproducibility of the silica coating process.

Example 2: Indium Oxine and Aluminium

A non-radioactive oxine stock solution without $^{111}$In was prepared from approved reagents. The active sub-batch was prepared from the same reagents but with sufficient $^{111}$In-indium chloride in 0.04 M HCl stock solution to give a reference activity of 0.1 mCi/ml.

For the active experiment, 10 P6 glass vials (ie. uncoated USP Type 1 glass vials) were each dispensed with 1 ml of radioactive stock solution. For the inactive experiment, approximately 390 P6 vials were each dispensed with 1 ml of oxine stock solution. All the vials were stoppered, oversealed and autoclaved in the approved manner. The active vials were subjected to RAC (radioactive concentration) determination, both initially and after standing for 1 day. The inactive vials were left for 3 days in the upright position.

The precipitate was harvested from the inactive P6 vials by the following procedure:

10 vials were decapped and decanted into a Sterilin container;
each vial was washed with 1 ml pyrogen-free water and the washings added to the Sterilin;
a further 10 vials were treated as above to create a second filled Sterilin;
the Sterilins were centrifuged at 2000 rpm for 10 minutes;
the supernatants were decanted and the above procedures repeated using the same Sterilin containers;
this cycle was repeated until all the inactive vial contents had been processed through the two Sterilins;
the precipitate in each Sterilin were transferred to separate tared vials and freeze-dried;
at the completion of freeze-drying, the vials were re-weighed.

Results:

(i) Active vials

The P6 vials gave a mean decrease in of $^{111}$In activity of 16.7% with a maximum of 38.7%. However, some of the P6 vials had initial RACs substantially below the expected value of 0.1 mCi/ml. The vials with the lower initial RACs tended subsequently to show the highest RAC drops.

(ii) Inactive vials

The processing of the inactive vials yielded a small but visible quantity of green precipitate in each Sterilin. The total yield of freeze-dried precipitate was 7.0 mg. $^{1}$H NMR and chromatographic analysis compared with an authentic sample of aluminium oxinate showed the precipitate to be predominantly aluminium oxinate.

Example 3: Oxine Ligand Loss

20 P6 glass vials were rinsed with water, drained and baked to depyrogenate. Each vial was then dispensed with an aqueous solution containing 50 μg of oxine, and the oxine content determined by HPLC at various time points. The results are shown in Table 2:

Table 2: Oxine Loss by HPLC

| Time Point | Oxine in Solution | Solution/ Suspension Al (ox)3 | Glass oxine | Glass Al(ox)3 | Recovery (%) |
|---|---|---|---|---|---|
| initial | 25.2 | 8.5 | 5.1 | 2.3 | 82 |
| reference | 20.1 | 26.7 | 3.1 | 6.0 | ~100 |
| expiry | 20.3 | 27.7 | 2.5 | 7.3 | ~100 |
| expiry +14 days | 19.0 | 16.4 | 6.6 | 5.7 | 95 |

HPLC System

Column: Hamilton PRP1, 150 x 4.1mm, $\mu$m particle size
Eluent A: 25 mM $KH_2PO_4$ adjusted to pH 10.3 - 10.5 with KOH
Eluent B: Acetonitrile

$$\textit{Gradient:}\quad 0\%\ B \xrightarrow{15\ min} 50\%\ B \xrightarrow{5\ min} 50\%\ B \xrightarrow{3\ min} 0\%\ B$$

Flow Rate: 1.75ml/min.
Injection volume: 20$\mu$g
Detection: UV at 254nm, path length 10mm

System control and data processing: Gilson 715 System Controller

The system was calibrated using 50 $\mu$g/ml solutions of oxine in water and aluminium oxinate in acetonitrile. Retention time of oxine was ca. 15 - 15.2 min, and Al(oxinate) ca. 17 min.

For solution/suspension measurements, samples of vial contents were taken directly and injected. The oxine and aluminium oxinate peaks were each integrated and the aluminium oxinate peak area normalised to give an oxine equivalent. The results were then scaled to give a total solution/suspension content. For measurements of oxine and aluminium oxinate adsorbed on the vial walls, the vial was decapped, drained and carefully rinsed with 0.2ml acetonitrile. 20 $\mu$g of the rinse solution was injected, the peaks integrated and the aluminium oxinate peak area normalised to give an oxine equivalent. Previous experiments had shown oxine losses to the vial stopper to be negligible.

Example 4

Freeze-dried kits using USP Type 1 glass vials (i.e. P6 uncoated vials) containing the following formulation were prepared:

| | |
|---|---|
| *meso*-DMSA | 1.0mg |
| $SnCl_2.2H_2O$ | 0.42mg |
| ascorbic acid | 0.7mg |
| inositol | 50.0mg |

These vials were reconstituted with 0.9% saline B.P. and set aside for 1 hour. A proportion (ca. 1 in 10) of the vials were found to contain aluminium levels of up to 2 $\mu$g/vial. Standing these vials for longer periods gave levels of up to 6 $\mu$g/vial of aluminium.

When the above lyophilised DMSA kit formulation vials were reconstituted with $^{99m}$Tc generator eluate (ie. $^{99m}$Tc-pertechnetate in saline) which was known to contain 1$\mu$g/ml aluminium ion, the product exhibited atypical elevated liver uptake in a rat biodistribution test. The atypical behaviour was reflected in reduced kidney: liver and spleen ratios in the range 5.9 to 7.7 at 4 hours post injection. In contrast, kits reconstituted with normal $^{99m}$Tc generator eluate give ratios of 13 to 20:1, i.e. significantly higher. Leached aluminium can thus be linked to poor product performance for a significant proportion of vials.

Example 5

A 100$\mu$g/ml solution of $CoCl_2.6H_2O$ was prepared by dissolving 0.100g $CoCl_2.6H_2O$ in water in a 1000ml volumetric flask and making up to volume. 2.0$cm^3$ portions of this solution were immediately dispensed into 6 of each of the following types of vials:

P6 standard glass,
Schott Type 1 Plus (silica-coated),

the vials were autoclaved, then allowed to cool, and the cobalt content assayed by UV. The results are given in Table 3, which includes values for the cobalt stock solutions for comparison.

Table 3: Cobalt Adsorption

| Vial | Vol added ($cm^3$) | Cobalt content ($\mu g/cm^3$) | Statistics |
|---|---|---|---|
| Stock ≠1 | 1.997 | 24.58 | |
| ≠2 | 1.998 | 24.41 | mean = 24.31 |
| ≠3 | 1.998 | 24.32 | max = 24.58 |
| ≠4 | 1.999 | 24.58 | min = 23.97 |
| ≠5 | 1.992 | 23.97 | |
| ≠6 | 1.995 | 24.03 | |
| P6 ≠1 | 1.976 | 13.87 | |
| ≠2 | 1.982 | 13.27 | mean = 14.61 |
| ≠3 | 1.985 | 16.52 | max = 17.84 |
| ≠4 | 1.978 | 17.84 | min = 12.80 |
| ≠5 | 1.982 | 13.40 | |
| ≠6 | 1.985 | 12.80 | |
| coated ≠1 | 1.986 | 25.21 | |
| ≠2 | 1.975 | 24.86 | mean = 24.49 |
| ≠3 | 1.979 | 23.81 | max = 25.21 |
| ≠4 | 1.983 | 24.82 | min = 23.81 |
| ≠5 | 1.987 | 24.40 | |
| ≠6 | 1.983 | 23.87 | |

**Claims**

1. A composition which comprises a radiopharmaceutical in a container which has a silica coating on the inner surface, **characterised in that** the radiopharmaceutical is a metal complex.

2. The composition of claim 1 where the radiopharmaceutical is a liquid or solution.

3. The composition of claims 1 or 2 where the metal of the metal complex is $^{111}$In or $^{99m}$TC.

4. The composition of claims 1 to 3 where the silica coating is deposited by a plasma chemical vapour deposition (PCVD) process.

5. The composition of claims 1 to 4 where the container is a glass vial with a closure.

6. A kit for the preparation of a sterile radiopharmaceutical metal complex which comprises a non-radioactive organic ligand composition in a container which has a silica coating on the inner surface.

7. The kit of claim 6 where the metal complex is a complex of the metal $^{99m}$Tc.

8. The kit of claims 6 or 7 where the non-radioactive organic ligand composition is lyophilised.

9. The kit of claims 6 to 8 where the silica coating is deposited by a plasma chemical vapour deposition (PCVD) process.

**Patentansprüche**

1. Zusammensetzung, umfassend ein Radiopharmazeutikum in einem Behälter, der eine Siliciumdioxid-Beschichtung auf der inneren Oberfläche aufweist, **dadurch gekennzeichnet, dass** das Radiopharmazeutikum ein Metallkomplex ist.

2. Zusammensetzung nach Anspruch 1, bei der das Radiopharmazeutikum eine Flüssigkeit oder Lösung ist.

3. Zusammensetzung nach Anspruch 1 oder 2, bei der das Metall des Metallkomplexes $^{111}$In oder $^{99m}$Tc ist.

4. Zusammensetzung nach den Ansprüchen 1 bis 3, bei der die Siliciumdioxid-Beschichtung durch einen Prozess der chemischen Abscheidung aus der Plasma-Gasphase (PCVD) abgeschieden worden ist.

5. Zusammensetzung nach den Ansprüchen 1 bis 4, bei der der Behälter ein Glasfläschchen mit einem Verschluss ist.

6. Kit für die Herstellung eines sterilen radiopharmazeutischen Metallkomplexes, umfassend eine nicht-radioaktive organische Liganden-Zusammensetzung in einem Behälter, die eine Siliciumdioxid-Beschichtung auf der inneren Oberfläche aufweist.

7. Kit nach Anspruch 6, bei dem der Metallkomplex ein Komplex des Metalls $^{99m}$Tc ist.

8. Kit nach Anspruch 6 oder 7, bei dem die nicht-radioaktive organische Liganden-Zusammensetzung lyophilisiert ist.

9. Kit nach den Ansprüchen 6 bis 8, bei dem die Siliciumdioxid-Beschichtung durch einen Prozess der chemischen Abscheidung aus der Plasma-Gasphase (PCVD) abgeschieden worden ist.

**Revendications**

1. Composition qui comprend un agent radiopharmaceutique dans un conteneur qui comporte un revêtement en silice sur la surface interne, **caractérisée en ce que** l'agent radiopharmaceutique est un complexe métallique.

2. Composition selon la revendication 1, dans laquelle l'agent radiopharmaceutique est un liquide ou une solution.

3. Composition selon les revendications 1 ou 2, dans laquelle le métal du complexe métallique est $^{111}$In ou $^{99m}$Tc.

4. Composition selon les revendications 1 à 3, dans laquelle le revêtement en silice est déposé par un procédé de dépôt chimique en phase vapeur activé au plasma (PCVD).

5. Composition selon les revendications 1 à 4, dans laquelle le conteneur est une éprouvette en verre avec un obturateur.

6. Kit pour la préparation d'un complexe métallique radiopharmaceutique stérile qui comprend une composition formant ligand organique non radioactif dans un conteneur qui comporte un revêtement en silice sur la surface interne.

7. Kit selon la revendication 6, dans lequel le complexe métallique est un complexe du métal $^{99m}$Tc.

8. Kit selon les revendications 6 ou 7, dans lequel la composition formant ligand organique non radioactif est lyophilisée.

9. Kit selon les revendications 6 à 8, dans lequel le revêtement en silice est déposé par un procédé de dépôt chimique en phase vapeur activé au plasma (PCVD).